# EUROPÄISCHE PATENTANMELDUNG

(11) **EP 4 610 342 A1**
(43) Veröffentlichungstag der Anmeldung: **03.09.2025**
(21) Anmeldenummer: 24160700.1
(22) Anmeldetag: 29.02.2024
(51) Int. Cl.: C12M 1/00

(54) **VORRICHTUNG UND ANLAGE ZUR AUFZUCHT UND WEITERVERARBEITUNG VON ALGEN IN EINEM GEWÄSSER SOWIE ZUR WASSERFILTRATION**

(71) Anmelder: Lempp, Alexander, 24148 Kiel (DE); Yan, On-Yee, 10967 Berlin (DE)
(72) Erfinder: Lempp, Alexander, 24148 Kiel (DE); Yan, On-Yee, 10967 Berlin (DE)
(74) Vertreter: Heinemeyer, Karsten

(57) **Zusammenfassung**

Beschrieben werden eine Vorrichtung (1) und eine Anlage zur Aufzucht und Weiterverarbeitung von Algen in einem Gewässer (10) sowie zur Wasserfiltration mit einer derartigen Vorrichtung. Die Vorrichtung (1) verfügt über einen Grundkörper und ein Tragelement (3), das eine zur Kultivierung von Algen (4) geeignete Vegetationsfläche (5) begrenzt und/oder an dem ein Vegetationselement (6) mit einer zur Kultivierung von Algen (4) geeigneten Vegetationsfläche (5) befestigbar oder befestigt ist. Der Grundkörper ist gemeinsam mit dem Tragelement (3) zumindest teilweise unter eine Wasseroberfläche des Gewässers (10) absenkbar.

Die Vorrichtung zeichnet sind dadurch aus, dass der Grundkörper eine Zentralstrebe (2) aufweist, durch die Zug- und Druckkräfte in Längsrichtung der Zentralstrebe (2) aufnehmbar sind, und dass das wenigstens eine Tragelemente (3) zumindest mittelbar an der Zentralstrebe (2) befestigt und zumindest bereichsweise beabstandet zu einer Außenfläche der Zentralstrebe (2) angeordnet ist.

## Beschreibung

Die Erfindung betrifft eine Vorrichtung sowohl zur Aufzucht und Weiterverarbeitung von Algen in einem Gewässer als auch zur Wasserfiltration sowie eine Anlage mit einer Mehrzahl derartiger Vorrichtungen. Die beschriebene Vorrichtung verfügt über einen Grundkörper und ein Tragelement, das ein Vegetationselement mit einer zur Kultivierung von Algen geeigneten Vegetationsfläche umgibt und/oder an dem ein Vegetationselement mit einer zur Kultivierung von Algen geeignete Vegetationsfläche befestigbar oder befestigt ist. Hierbei ist der Grundkörper gemeinsam mit dem Tragelement zumindest teilweise unter eine Wasseroberfläche des Gewässers absenkbar.

Allgemein handelt es sich bei Algen um eukaryotische Lebewesen, die im Wasser leben und Photosynthese betreiben. Sie sind in vielen Lebensmitteln und Kosmetika enthalten und es wird derzeit davon ausgegangen, dass Algen einen nicht unerheblichen Beitrag zur Nahrungsmittelversorgung der wachsenden Weltbevölkerung liefern können. Algenfarmen existieren in unterschiedlichen küstennahen Gebieten auf der Erde, bevorzugt in Gezeitenrevieren, insbesondere in Lagunen oder küstennahen Flachwasserregionen, wobei die Hauptanbaugebiete derzeit noch in Asien liegen. Nichtsdestotrotz findet auch in deutschen Gewässern Algenanbau statt, hier insbesondere in den Wintermonaten.

Grundsätzlich sind aus dem Stand der Technik zwei unterschiedliche Lösungsansätze bekannt, die für die gezielte Algenaufzucht verwendet werden, nämlich einerseits der Anbau im Meer, andererseits aber auch der Anbau in speziellen Anbauvorrichtungen an Land.

In diesem Zusammenhang ist aus der DE 198 35 656 A1 ein Verfahren sowie eine Vorrichtung zur Algenkultivierung bekannt, bei dem das Algenwachstum in einer feuchten Atmosphäre innerhalb eines Kultivierungsraumes erfolgt. Hierbei verfügt der Kultivierungsraum über eine Verankerungseinrichtung zur Befestigung der zu kultivierenden Algen und über eine Flüssigkeitszufuhr, durch die ein Nährmedium in Form eines Aerosols oder als Sprühstrahl in den Kultivierungsraum eingebracht wird. In der beschriebenen Anlage können die Wachstumsbedingungen für die Algen, etwa die Temperatur, das Nährmedium und die Beleuchtung bedarfsgerecht eingestellt werden. Nachteilig an dem beschriebenen System ist allerdings der vergleichsweise große apparative Aufwand, der Energie-, Wasser- sowie Platzbedarf an Land.

Im Vergleich zu den Anlagen, die für die kontrollierte Algenaufzucht an Land, beispielsweise für die kosmetische und pharmazeutische Industrie, verwendet werden, zeichnen sich Algenfarmen im Meer durch einen sehr einfachen Aufbau aus. Üblicherweise werden an langen Leinen oder Netzen, die teilweise spezielle Haltesysteme aufweisen, Algensetzlinge befestigt oder aber Leinen oder Netze mit Algensporen imprägniert und gemeinsam mit diesen im Meer ausgebracht. Nach einigen Monaten werden die Leinen oder Netze mit den zwischenzeitlich gewachsenen Algen von Schiffen aus eingeholt und die Algen mit Hilfe von geeigneten Maschinen von den Leinen oder Netzen abgeschnitten. Problematisch an diesem Verfahren zur Algenaufzucht ist vor allem, dass die Leinen oder Netze Kunststoffmaterial aufweisen, teilweise nur einmal verwendet werden und in den Meeren eine große Anzahl von Leinen oder Netzen, sogenannte Geisternetze, verbleibt, sodass die Umwelt hierdurch stark belastet wird. Ebenso ist nicht auszuschließen, dass sich während der Ernte der Algen, also wenn diese von den Leinen oder Netzen abgeschnitten werden, Kunststoffe in die geernteten Algen eingetragen werden, was letztendlich zu einer ungewünschten Verschmutzung dieses Naturprodukts führt. Im Weiteren ist es bei dieser Form des Algenanbaus nicht oder nur sehr schwer möglich, während der Wachstumsphase die Position der zu kultivierenden Algen zu verändern, beispielsweise um bessere Wachstumsbedingungen erreichen zu können. Darüber hinaus ist zu beachten, dass viele Algensorten nicht an Leinen gezüchtet werden können, da die Oberfläche der Leinen für die Aufzucht dieser Algen ungeeignet ist.

Im Weiteren ist bei den bekannten Anlagen zur Algenaufzucht der Aufwand für das Ausbringen und Ernten der Algen erheblich. Aufgrund der konstruktiven Gestaltung und Anordnung der bekannten Anlagen in dem jeweiligen zur Algenaufzucht vorgesehenen Gewässer, sind diese ferner nur schwer an sich ändernde Gegebenheiten im Gewässer und/oder in Bezug auf eine Veränderung der Produktionsleistung anpassbar.

Ausgehend von den aus dem Stand der Technik bekannten Lösungen zur agrarindustriellen Algenaufzucht sowie den zuvor geschilderten Problemen liegt der Erfindung die Aufgabe zu Grunde, eine Vorrichtung sowie eine Anlage mit einer Mehrzahl derartiger Vorrichtungen bereitzustellen, durch die auf vergleichsweise einfache und effektive Weise eine nachhaltige, insbesondere umweltverträgliche, nur wenig in die Gewässerökologie eingreifende Aufzucht von Algen ermöglicht wird, wobei insbesondere eine besonders geeignete Versorgung der aufzuziehenden Algen mit Wasser, Nährstoffen und Licht erreicht werden soll. Ferner sollte eine Möglichkeit geschaffen werden, die Aufzucht von Algen bzw. deren Produktion leicht zu skalieren, um in Abhängigkeit der zur Verfügung stehenden Gewässerfläche, Gewässertiefe und der Wasserqualität die Größe und damit die Produktionsleistung einer Anlage zur Algenaufzucht vergleichsweise einfach an die jeweiligen Verhältnisse anpassen zu können. Im Weiteren sollte eine Effizienzsteigerung bei der Aufzucht von Algen im Vergleich zu bekannten Systemen ermöglicht und damit eine Steigerung des in einem Zeitraum erzielbaren Algenertrags erreichbar sein. Gleichzeitig sollten sich die einzelnen Komponenten einer Vorrichtung zur Algenaufzucht durch einen verhältnismäßig einfachen Aufbau und dennoch die erforderliche Robustheit auszeichnen und sich unter wirtschaftlichen Gesichtspunkten sinnvoll herstellen und montieren lassen.

Darüber hinaus sollte die anzugebende Vorrichtung eine möglichst einfache Handhabung beim Ausbringen in und Einholen aus einem Gewässer und bei der Verankerung im Gewässeruntergrund gewährleisten.

Im Weiteren wird ein Aspekt darin gesehen, eine Vorrichtung zur Aufzucht von Algen zu schaffen, die sich mit möglichst geringem Aufwand in bestehende Anlagen integrieren lässt, wobei insbesondere eine Befestigung der zur Aufzucht von Algen vorgesehenen Vorrichtungen sowohl an im Wasser angeordneten Schwimmleinen als auch an einer in den Gewässeruntergrund eingebrachten Verankerung möglich sein soll.

Die zuvor beschriebene Aufgabe wird mit einer Vorrichtung gemäß Anspruch 1 sowie mit einer eine Anlage nach Anspruch 15 gelöst. Vorteilhafte Ausführungsformen der Erfindung sind Gegenstand der abhängigen Ansprüche und werden in der folgenden Beschreibung unter teilweiser Bezugnahme auf Figuren näher erläutert.

Die Erfindung betrifft eine Vorrichtung zur Aufzucht und Weiterverarbeitung von Algen in einem Gewässer sowie zur Wasserfiltration mit einem Grundkörper und einem Tragelement, das ein Vegetationselement mit einer zur Kultivierung von Algen geeigneten Vegetationsfläche zumindest bereichsweise umgibt und somit einen Bereich, in dem sich zumindest eine Vegetationsfläche befindet, begrenzt und/oder an dem ein Vegetationselement mit einer zur Kultivierung von Algen geeigneten Vegetationsfläche befestigbar oder befestigt ist. Hierbei ist es wesentlich, dass der Grundkörper und das Tragelement derart ausgeführt sind, dass diese gemeinsam wenigstens für einen gewissen Zeitraum zumindest teilweise unterhalb eine Wasseroberfläche des Gewässers absenkbar sind. Die erfindungsgemäß ausgeführte Vorrichtung zeichnet sich dadurch aus, dass der Grundkörper eine Zentralstrebe aufweist, durch die Zug- und Druckkräfte in Längsrichtung der Zentralstrebe aufnehmbar sind, und dass das wenigstens eine Tragelemente an der Zentralstrebe befestigt und zumindest bereichsweise beabstandet zu einer Außenfläche der Zentralstrebe angeordnet ist. Ein wesentliches Element der Erfindung stellt somit die Zentralstrebe dar, die wenigsten teilweise ein festes Material, wie etwa Metall, Stahl und/oder Kunststoff, aufweist, sodass in Längsrichtung der Zentralstrebe Druck- und Zugkräfte aufgenommen werden können, ohne dass es, zumindest über einen vergleichsweise großen Kraftbereich zu einer Verformung der Zentralstrebe kommt. Die Zentralstrebe stellt als Grundkörper ein wesentliches Bauelement der erfindungsgemäßen Vorrichtung dar und ist auf vorteilhafte Weise derart gestaltet, dass diese während des bestimmungsgemäßen Gebrauchs zur Aufzucht von Algen im Wesentlichen vertikal in dem umgebenden Gewässers angeordnet ist, also die Längsachse der Zentralstrebe zumindest nahezu senkrecht zum Gewässeruntergrund angeordnet ist, auch wenn es verständlich ist, dass sich die Neigung der Längsachse der Zentralstrebe relativ zum Gewässeruntergrund aufgrund von Strömungen, Windeinfluss und/oder Wellen bzw. Seegang verändern kann. Mit dieser Zentralstrebe ist zumindest mittelbar wenigstens ein Tragelement verbunden, das derart ausgeführt ist, dass ein Vegetationselement mit einer zur Aufzucht von Algen geeigneten Vegetationsfläche an dem Tragelemente befestigt oder befestigbar ist. Ebenso ist es denkbar, dass das Tragelement, beispielsweise in Form eines Netzes, Korbes oder Käfigs, ein Vegetationselement mit einer zur Aufzucht von Algen geeigneten Vegetationsfläche zumindest abschnittsweise umgibt. Das verwendete Tragelemente ist zumindest bereichsweise beabstandet zur Zentralstrebe angeordnet, sodass zumindest Bereiche oder Teile des wenigstens einen Tragelements in einem Abstand zur Zentralstrebe angeordnet sind.

Unter dem Begriff Algen werden grundsätzlich ein- oder mehrzellige pflanzenartige Lebewesen zusammengefasst, die in wässrigen Lebensräumen als Primärproduzenten die erste Stufe der Nahrungskette bilden. Algen benötigen zu ihrem Wachstum Licht, Kohlenstoffdioxid oder Hydrogencarbonat, Mineralstoffe und Nährstoffe. Sie vermehren sich üblicherweise durch Zellteilung oder vegetativ. Pflanzliche Algen vermehren sich über Sporen, die sich in einem Gewässer befinden. Bevorzugt werden mit der erfindungsgemäßen Vorrichtung Algen der Gattung Ulva aufgezogen. Hierbei handelt es sich um eine Gattung vielzelliger Grünalgen, die mit etwa 130 Arten weltweit in den Meeren verbreitet ist. Ihr Thallus besteht aus zwei Zellschichten und ist röhrig bis blattartig gestaltet, wobei sich die Arten morphologisch stark unterscheiden können.

Gemäß einer besonderen Ausgestaltung der Erfindung ist vorgesehen, dass das Tragelement wenigstens eine Strebe, eine Leine, eine Kette und/oder ein Seil aufweist, deren oder dessen Längsachse parallel oder geneigt, insbesondere in einem spitzen Winkel zur Außenumfangsfläche der Zentralstrebe angeordnet ist. Ein entsprechend ausgebildetes Tragelemente dient der Anbringung oder Befestigung eines Vegetationselements, das eine zur Aufzucht von Algen geeignete Vegetationsfläche aufweist. Hierbei verfügt die an oder auf einem Vegetationselement vorgesehene Vegetationsfläche über eine Schicht oder ist als Beschichtung auf das Vegetationselement ausgeführt, die Algensporen, Algensetzlinge und/oder wenigstens einen ein Algenwachstum begünstigenden Nährstoff aufweist.

In einer weiteren speziellen Ausgestaltung der Erfindungen wird das Tragelement aus wenigstens zwei Modulen und/oder Segmenten gebildet. Die entsprechenden Module oder Segmente können hierbei bedarfsgerecht zumindest mittelbar an der Zentralstrebe befestigt werden. Ebenso ist es denkbar, dass wenigstens zwei Module und/oder Segmente über geeignete Befestigungsmittel miteinander verbunden. Vorzugsweise erfolgt die Verbindung der Module oder Segmente an der Zentralstrebe und/oder untereinnander derart, dass die Verbindung werkzeuglos herstellbar und lösbar ist.

Ein modul- oder segmentartiger Aufbau eines Tragelementes hat vor allem den Vorteil, dass sich die Vorrichtung zur Aufzucht von Algen mit einer derartigen Tragelement sowie eine Anlage aus einer Mehrzahl derartiger Vorrichtungen vergleichsweise einfach skalieren lässt, also die mit einer Vorrichtung bzw. einer Anlage erzielbare Algenproduktion in Abhängigkeit der Anzahl der verwendeten Module und/oder Segmente bedarfsgerecht gesteigert oder verringert werden kann. Ebenso lässt sich die Vorrichtung an die jeweils vorhandenen Gewässergegebenheiten, wie etwa die Wassertiefe und die Strömungseigenschaften anpassen.

Eine weitere Ausführungsform der Erfindung sieht vor, dass das Vegetationselement als Schicht auf dem Tragelement ausgeführt ist. Ein derartiges Vegetationselement in Form einer Schicht oder Beschichtung bildet auf vorteilhafte Weise zumindest bereichsweise auf der Ober- oder Mantelfläche eines Tragelements eine Vegetationsfläche aus, die geeignet für die Aufzucht von Algen in einem Gewässer ist. Vorzugsweise ist es in diesem Zusammenhang denkbar, dass die Beschichtung im Bereich ihrer Oberfläche über Sporen, Nährstoffe und/oder Mineralstoffe verfügt, die die Aufzucht von Algen in einem Gewässer ermöglichen und/oder zumindest begünstigen.

Im Weiteren ist es denkbar, dass das Tragelement netz- und oder maschenartig ausgebildet ist. Ein derartiges Tragelement ist wiederum derart ausgeführt und wenigstens mittelbar mit der Zentralstrebe verbunden, dass zumindest ein Vegetationselement von dem Tragelement begrenzt und/oder ein Vegetationselement mit seiner Vegetationsfläche auf dem Tragelement angeordnet oder an diesem befestigt ist. Von besonderem Vorteil ist es, dass ein netz- oder maschenartig ausgebildetes Tragelement auf seiner Oberfläche zumindest bereichsweise ein Vegetationselement in Form einer Beschichtung aufweist, die als Vegetationsfläche und somit der Aufzucht von Algen in einem Gewässer dient.

Eine weitere spezielle Ausführungsformen der Erfindung sieht vor, dass das Tragelement wenigstens abschnittsweise in Form eines Hohlzylinders, Hohlkegelstumpfs und/oder Hohlquaders ausgebildet. In diesem Zusammenhang ist es ferner denkbar, dass das Tragelement über eine geschlossene Oberfläche verfügt oder wenigstens teilweise aus Streben oder einem Netz gebildet wird. Im Weiteren ist es denkbar, dass auf der äußeren Oberfläche, insbesondere der Mantelfläche, eines entsprechend ausgebildeten Tragelements wenigstens abschnittsweise ein Vegetationselement in Form einer Beschichtung, auf der eine Vegetationsfläche angeordnet ist, vorgesehen ist. Alternativ oder in Ergänzung ist es denkbar, dass ein in Form eines Hohlzylinders, Hohlkegelstumpfs und/oder Hohlquaders ausgebildet Tragelement ein Vegetationselement mit seiner Vegetationsfläche begrenzt. Das Tragelement bildet in diesem Fall eine Art Korb oder Käfig, in dem die Vegetationselemente mit ihren Vegetationsflächen frei in dem für die Algenaufzucht vorgesehenen Gewässer schwimmen können. In diesem Zusammenhang sieht eine ganz spezielle Ausgestaltung der Erfindung vor, dass das Tragelement in Form eines Hohlzylinders, Hohlkegelstumpfs oder Hohlquaders ausgebildet ist, in dessen Innenraum zerkleinerte Algenfragmente frei schwimmen. Das Tragelement umgibt somit den für die Aufzucht von Algen vorgesehenen Bereich oder Raum. Es ist ferner denkbar, dass während des Prozesses der Algenaufzucht kleine Algenfragmente und/oder vergleichsweise kleine Vegetationselemente, etwa Leinenstücke, auf deren Oberfläche sich eine Vegetationsfläche zur Aufzucht von Algen befindet, im Inneren des wie zuvor beschrieben ausgebildeten Tragelements schwimmend angeordnet sind.

Eine weitere Ausführungsform Erfindung sieht vor, dass das Tragelement ein Plattenelement oder eine Mehrzahl von Plattenelementen aufweist, das zumindest nahezu in einer Ebene liegt, die parallel oder in einem spitzen Winkel geneigt zur Längsachse der Zentralstrebe angeordnet ist. Ein derartiges Plattenelement ist auf vorteilhafte Weise an der Zentralstrebe befestigt, wobei es denkbar ist, dass geeignete Befestigungsmittel vorgesehen sind, um eine einfach, insbesondere werkzeuglos herstell- und lösbare Verbindung zwischen dem plattenförmigen Tragelement und der Zentralstrebe zu realisieren. Vorzugsweise ist auf der Oberfläche eines derartigen plattenförmigen Tragelementes ein schichtförmiges Vegetationselement mit einer Vegetationsfläche vorgesehen, die wiederum die geeignete Aufzucht von Algen in einem Gewässer gewährleistet. In diesem Zusammenhang wurde überraschend festgestellt, dass sich besonders Plattenelemente mit einer vergleichsweise glatten Oberfläche zur Aufzucht von Algen in einem Gewässer eignen. In diesem Zusammenhang ist es auf vorteilhafte Weise denkbar, dass das Tragelement ein Plattenelement aufweist, das aus einem Metallblech, einem Kunststoffmaterial und/oder aus Glas gefertigt ist. Eine derart glatte Oberfläche bildet hierbei günstige Bedingungen für die schnelle und effektive Aufzucht von Algen.

Im Weiteren ist es denkbar, dass die Plattenelement nicht eben, sondern mit einer speziellen Kontur ausgebildet sind, also verformt ist. So ist es auf vorteilhafte Weise denkbar, dass ein Plattenelement wenigstens bereichsweise gebogen, aufgerollt, geknickt und/oder gefaltet ist. Dies bietet den Vorteil, dass sich auf vergleichsweise kleinem Raum eine möglichst große Vegetationsfläche zur Verfügung stellen lässt. Gemäß einer weiteren speziellen Gestaltung ist vorgesehen, dass das Tragelement zwischen der Zentralstrebe und einer umgebenden, bevorzugt hohlzylinder- oder hohlquaderförmig ausgebildeten Abschirmeinheit angeordnet ist. Eine derartige Abschirmeinheit, die vorzugsweise wenigstens mittelbar mit der Zentralstrebe verbunden ist, bietet die Möglichkeit, dass auch das Tragelement wenigstens bereichsweise an der Abschirmeinheit befestigt ist, sodass insbesondere die Stabilität und Verwindungssteifigkeit der aus Zentralstrebe, Tragelement und Abschirmeinheit gebildeten Baueinheit im Vergleich zu anderen Lösungen erhöht wird. Eine derartige Abschirmeinheit kann als Hohlzylinder, Hohlquader oder aber als sonstiger rohrförmiger Körper mit ovalem oder mehreckigem Querschnitt ausgeführt sein. Die Abschirmeinheit kann ferner über eine geschlossene oder eine wenigstens teilweise offene, insbesondere maschen-, gitter- oder netzartige, Oberfläche verfügen. Ebenso ist es denkbar, dass das Abschirmeinheit aus einer Mehrzahl von Streben gebildet wird, die in Umfangsrichtung und/oder in Längsrichtung der Zentralstrebe angeordnet sind.

Das wenigstens eine Tragelement, bevorzugt eine Mehrzahl von Tragelementen mit Vegetationselement und Vegetationsfläche ist auf vorteilhafte Weise zwischen der Abschirmeinheit und der Zentralstrebe angeordnet.

In einer besonderen Ausführungsformen der Erfindung verfügt die Zentralstrebe über wenigstens eine Leuchteinheit. Denkbar ist in diesem Zusammenhang, dass die Eigenschaften des emittierten Lichts, insbesondere die Farbe, Wellenlänge und/oder ein Wellenlängenbereich, beispielsweise in Abhängigkeit der aufzuziehenden Algenart, der Tageszeit, der herrschenden Lichtverhältnisse und/oder sonstiger Umwelteinflässe, gezielt auswählbar oder einstellbar ist. Vorzugsweise sieht eine besondere Ausführungsform der Erfindung dass die Leuchteinheit Licht mit einer Wellenlänge im Bereich von 100 bis 308 nm, also ultraviolettes Licht, emittiert. Bevorzugt ist die verwendete Leuchteinheit als LED ausgeführt.

Im Weiteren ist es von Vorteil, wenn innerhalb der Zentralstrebe ein Energiespeicher zur Versorgung der Leuchteinheit und/oder einer anderen Komponente, wie etwa eines Sensors oder einer Gasdosiereinheit, mit der erforderlichen elektrischen Energie angeordnet ist.

Alternativ oder in Ergänzung ist es denkbar, dass eine Einheit zur Erzeugung von elektrischer Energie mit der Zentralstrebe verbunden ist. Hierbei kann es sich um eine während des Betriebs unterhalb der Wasseroberfläche des umgebenden Gewässers angeordnete Einheit, die Strömungs- und/oder Wellenenergie nutzt, oder aber um ein oberhalb der Wasseroberfläche angeordnetes Element, wie ein Windrad oder eine Windturbine oder insbesondere um ein Fotovoltaikmodul, handeln. Die erzeugte elektrische Energie lässt sich auf vorteilhafte Weise direkt von elektrischen Verbrauchern nutzen oder in einem Energiespeicher speichern. Bei Einsatz eines Fotovoltaikmoduls ist es denkbar, während des Tages elektrische Energie zu erzeugen und diese in dem Energiespeicher zu speichern, sodass diese beispielsweise in Nachtstunden oder während Schlechtwetterperioden für eine Leuchteinheit verwendbar ist, um zumindest zeitweise die aufzuziehenden Algen zu beleuchten.

Eine weitere Ausführungsform der Erfindung sieht vor, dass eine Leuchteinheit vorgesehen ist, die wenigstens einen Lichtwellenleiter, zum Beispiel mit zumindest einer Glasfaser aufweist, über den Licht zu einer Emissionsfläche leitbar ist. In diesem Zusammenhang ist es weiterhin denkbar, dass über den Lichtwellenleiter zumindest zeitweise oberhalb der Wasseroberfläche, etwa mit einer Parabolrinne oder einem Parabolspiegel, aufgefangenes Sonnenlicht zu einer Emissionsfläche geleitet wird. Um eine geeignete Lichtleitung zu erreichen, ist es ferner denkbar, geeignete Optikelemente, insbesondere zur Umlenkung und/oder Bündelung von Lichtstrahlen zu verwenden. Gemäß der zuvor beschriebenen Ausführungsformen ist es somit wesentlich, dass die Zentralstrebe über wenigstens eine Einheit oder ein Bauelement verfügt, die zumindest Teil einer Leuchteinheit sind, mit der bei Dunkelheit und/oder zusätzlich zu dem auf die Wasseroberfläche eingestrahlten Sonnenlicht eine Beleuchtung der in der unmittelbaren Umgebung der Zentralstrebe gezüchteten Algen erfolgen kann.

Im Weiteren ist es von Vorteil, wenn eine erfindungsgemäß ausgeführte Vorrichtung zur Aufzucht von Algen in einem Gewässer über Mittel zur wenigstens mittelbaren Verankerung in einem Gewässeruntergrund verfügt. Denkbar ist in diesem Zusammenhang, dass an einem während des Betriebs der Vorrichtung dem Gewässeruntergrund zugewandten unteren Ende der Zentralstrebe eine Öse, ein Ring, ein Haken und/oder eine Klampe vorgesehen ist, mit der die Zentralstrebe zumindest mittelbar über eine Leine und/oder eine Kette an einem Grundanker befestigbar ist. Alternativ oder Ergänzung ist denkbar, dass wenigstens ein Befestigungselement vorgesehen ist, um die Zentralstrebe ebenfalls zumindest mittelbar mit einer im Gewässer angeordneten Leine und/oder Kette, insbesondere einer Schwimmleine zu verbinden. Denkbar ist in diesem Zusammenhang, dass eine erfindungsgemäß ausgebildete Vorrichtung an einer bereits im Gewässer, etwa kurz unterhalb der Wasseroberfläche verlaufenden Leine befestigt wird. Auf diese Weise ist es möglich, die erfindungsgemäße Vorrichtung zur Aufzucht von Algen vergleichsweise einfach in bereits bestehende Anlagen zur Algenaufzucht zu integrieren. Die jeweils erforderlichen Befestigungselemente sind Teil der Zentralstrebe oder an dieser befestigt.

Gemäß einer weiterer speziellen Ausgestaltung der Erfindung ist vorgesehen, dass die Zentralstrebe ein Funktionsmodul oder eine Mehrzahl geeigneter Funktionsmodule aufweist, die jeweils in die Zentralstrebe integriert oder an dieser befestigt sein können. Bei einem derartigen Funktionsmodul kann es sich insbesondere um ein Fotovoltaikmodul, einen Kohlenstoffdioxidspeicher, eine Dosiereinheit zur Ausbringung von Kohlenstoffdioxid in das Gewässer, einen Sensor, etwa einen Helligkeits-, Temperatur- und/oder Gassensor, eine Datensende- und/oder Empfangseinheit, einen Datenspeicher und/oder eine Datenverarbeitungseinheit handeln. Deutlich wird hierbei, dass es sich bei der Zentralstrebe gemäß der Erfindung um ein Bauteil handeln kann, dass nicht nur als statisches Element Zug- und Druckkräfte aufnimmt, sondern auch Platz und Raum zur bedarfsgerechten Aufnahme von optischen, elektrischen und/oder elektronischen Elementen und/oder von Elementen zur Gasspeicherung und/oder Gasausbringung, insbesondere für Kohlenstoffdioxid, bereitstellt.

Die erfindungsgemäße Vorrichtung kann grundsätzlich freischwimmend oder am Untergrund oder anderen Elementen befestigt für die Aufzucht von Algen in einem Gewässer sowie allein oder gemeinsam mit anderen, vorzugsweise baugleichen Vorrichtungen genutzt werden. Von Vorteil ist es, wenn eine Mehrzahl erfindungsgemäßer Vorrichtungen in ein Gewässer ausgebracht wird, wobei die Mehrzahl von Vorrichtungen vorzugsweise zumindest mittelbar mit dem Gewässeruntergrund verbunden wird. Die Erfindung betrifft insoweit auch eine Anlage zur Algenaufzucht in einem Gewässer mit einer Mehrzahl von Vorrichtungen, die gemäß der Erfindung oder wenigstens einer der zuvor beschriebenen speziellen Ausführungsformen der Erfindung gestaltet sind.

Vorzugsweise ist eine derartige Anlage wenigstens teilweise in einem Gewässeruntergrund verankert und/oder die einzelnen Vorrichtungen sind zumindest zum Teil miteinander verbunden.

Generell ist es denkbar, dass eine erfindungsgemäß ausgeführt Vorrichtung und/oder eine Anlage gemäß der Erfindung zur Aufzucht von Algen, insbesondere aus Sporen, zur Weiterverarbeitung von Algen, um wenigstens eine Eigenschaft einer Alge, wie etwa Größe, Gewicht, Dichte, Blattmasse, Farbe und/oder Struktur zu verändern und/oder zur Filtration von Wasser verwendet wird. Es ist somit denkbar, dass eine Vorrichtung und/oder Anlage derart dimensioniert, ausgeführt und/oder angelegt wird und/oder die Art der aufzuziehenden Algen derart gewählt wird, dass gezielt Inhaltsstoffe aus einem Gewässer, in dem sich die Vorrichtung und/oder Anlage befindet, gefiltert werden und/oder sich eine Zusammensetzung des Gewässers auf gewünschte Weise ändert, sich beispielsweise der Sauerstoffgehalt auf vorteilhafte Weise erhöht. Insofern ist die erfindungsgemäße Vorrichtung sowie eine Anlage gemäß der Erfindung generell auch zur Filtration und/oder Aufbereitung des Wassers eines Gewässers geeignet.

Im Folgenden wird die Erfindung ohne Beschränkung des allgemeinen Erfindungsgedankens anhand von Ausführungsbeispielen unter Bezugnahme auf Figuren näher erläutert. Dabei zeigen:
- Fig. 1:: Darstellung unterschiedlicher Ausführungsformen eines Tragelements für die erfindungsgemäße Vorrichtung;
- Fig. 2:: Darstellung unterschiedlicher Ausführungsformen eines Vegetationselements für die erfindungsgemäße Vorrichtung;
- Fig. 3:: Darstellung unterschiedlicher Ausführungsformen einer Abschirmeinheit für die erfindungsgemäße Vorrichtung;
- Fig. 4:: Darstellung zweier Anlagen mit einer Mehrzahl erfindungsgemäßer Vorrichtungen;
- Fig. 5:: schräge Draufsicht auf eine Anlage mit einer Mehrzahl erfindungsgemäßer Vorrichtungen;
- Fig. 6:: Darstellung einer Anlage mit einer Mehrzahl erfindungsgemäßer, an einer Schwimmleine befestigter Vorrichtungen;
- Fig. 7:: Darstellung von drei unterschiedlichen Ausführungsformen eines Tragelements für eine erfindungsgemäße Vorrichtung sowie
- Fig. 8:: Darstellung einer spezielle Zentralstrebe mit Funktionseinheiten für eine erfindungsgemäße Vorrichtung.

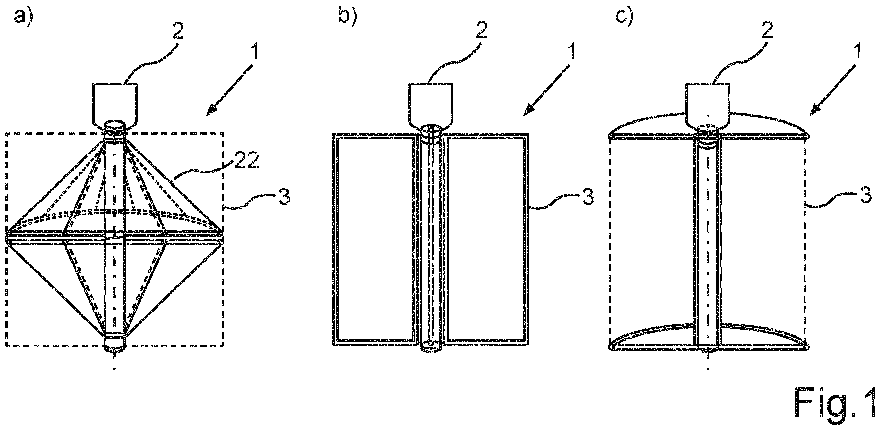

Fig. 1 zeigt in den Teilansichten a), b) und c) spezielle Ausgestaltungen einer erfindungsgemäßen Vorrichtung 1 zur Aufzucht von Algen 4 in einem Gewässer 10. Wesentlich an den drei unterschiedlichen in Fig. 1 gezeigten Ausführungsformen ist, dass die gezeigten Vorrichtungen 1 jeweils über eine Zentralstrebe 2 verfügen, an der unterschiedlich ausgebildete Tragelemente 3 befestigt sind. An den Tragelementen 3 sind, wie in Fig. 1a) und 1b) zu erkennen ist, wiederum Vegetationselemente 6 mit Vegetationsflächen 5 zur Aufzucht von Algen 4 befestigt oder befestigbar. In der Ausführungsform gemäß Fig. 1c) ist das Tragelement 3 in Form eines wasserdurchlässigen Käfigs oder Korbs ausgebildet, in dessen Innenraum während der Aufzucht der Algen 4 als Fragmente, hier entweder imprägnierte Seilfragmente oder Algenfragmente, ausgebildete Vegetationselemente 6 mit entsprechenden Vegetationsflächen 5 angeordnet sind.

In allen in Fig. 1 gezeigten Fällen verfügt die Zentralstrebe 2 nicht nur über geeignete Befestigungselemente, insbesondere mit einer wassergeeigneten Schnellkupplung, mittels denen die Tragelemente 3 mit der Zentralstrebe 2 verbunden sind, sondern es sind jeweils auch spezielle Befestigungselemente 21 vorgesehen, die eine zumindest mittelbare Befestigung der Zentralstrebe 2 mit den übrigen daran befestigen Bauelementen an einer Verankerung 11 im Gewässergrund ermöglichen.

In der Teilansicht a) der Fig. 1 ist eine Vorrichtung 1 zur Aufzucht von Algen in einem Gewässer 10 dargestellt, die über ein Tragelement 3 mit zwei kegelstumpfartig geformten Segmenten, wobei die Mantelflächen der beiden Module lediglich von einzelnen, gegenüber der Längsachse der Zentralstrebe 2 geneigten Streben 22 gebildet wird, die gemeinsam eine kegelstumpfförmige Mantelfläche aufspannen. Die beiden baugleichen Module sind derart angeordnet, dass die Seiten mit dem jeweils größeren Umfang einander zugewandt sind. Das aus zwei baugleichen Modulen oder Segmenten gebildete Tragelement 3 bietet die Möglichkeit, an den einzelnen Streben 22 des Tragelements 3 als Vegetationselemente 6 dienende Leinen oder Ketten, auf deren Oberflächen Vegetationsflächen 5 angeordnet sind, zu befestigen. Insbesondere ist es möglich, als Vegetationselemente 6 dienende Seile oder Ketten über die einzelnen Streben 22 der beiden Module des Tragelements 3 zu wickeln bzw. zu spannen.

Fig. 1b) zeigt eine weitere spezielle Ausführungsform der erfindungsgemäß ausgeführten Vorrichtung 1 zur Aufzucht von Algen 4. Die dargestellte Vorrichtung 1 verfügt wiederum über eine Zentralstrebe 2, an der gemäß dieser Ausführungsform allerdings zwei plattenförmige Tragelemente 3 befestigt sind. Auf diese Tragelemente 3 sind Vegetationselemente 6 in Form von Beschichtungen aufgebracht, deren Oberflächen die Funktion von Vegetationsflächen 5 haben. Die Vegetationsflächen 5 verfügen insbesondere über Algensporen, Nährstoffe und/oder Mineralstoffe, die geeignet zur Aufzucht von Algen 4 sind. Wesentlich für das verwendete plattenförmige Tragelement 3 ist, dass die verwendeten Platten eine ebene und glatte Oberfläche aufweisen. Bevorzugt wird eine Platte aus Glas oder Metall oder einem für die Aufzucht von Algen 4 geeigneten, umweltverträglichen Kunststoff verwendet.

Fig. 1c) zeigt eine weitere spezielle Ausführungsform der erfindungsgemäßen Vorrichtung 1 zur Aufzucht von Algen 4. Gemäß dem in Fig. 1c) gezeigten Ausführungsbeispiel verfügt die Vorrichtung 1 wiederum über eine Zentralstrebe 2 als Grundkörper. In diesem Fall ist an der Zentralstrebe 2 ein hohlzylinderförmiges Tragelement 3, das eine maschennetzartige Mantelfläche mit entsprechenden Öffnungen aufweist, befestigt. Alternativ kann ein hohlzlinderförmiges Tragelement 3 mit geschlossener Mantelfläche verwendet werden.

Bei der in Fig. 1c) gezeigten Ausführungsform einer erfindungsgemäßen Vorrichtung ist es möglich, im Inneren des Hohlzylinders zerkleinerte Algenfragmente anzuordnen, die sich zwischen der Zentralstrebe und der Mantelfläche des maschennetzartigen Tragelementes freischwimmend bewegen können. Die Bewegungsfreiheit wird hierbei durch das maschennetzartige Tragelement 3 begrenzt. Denkbar ist in diesem Zusammenhang, dass der Raum, in dem die Fragmente angeordnet sind, auch nach oben und nach unten durch Abschlussdeckel begrenzt wird, die bevorzugt wiederum aus einem Maschennetz gebildet werden.

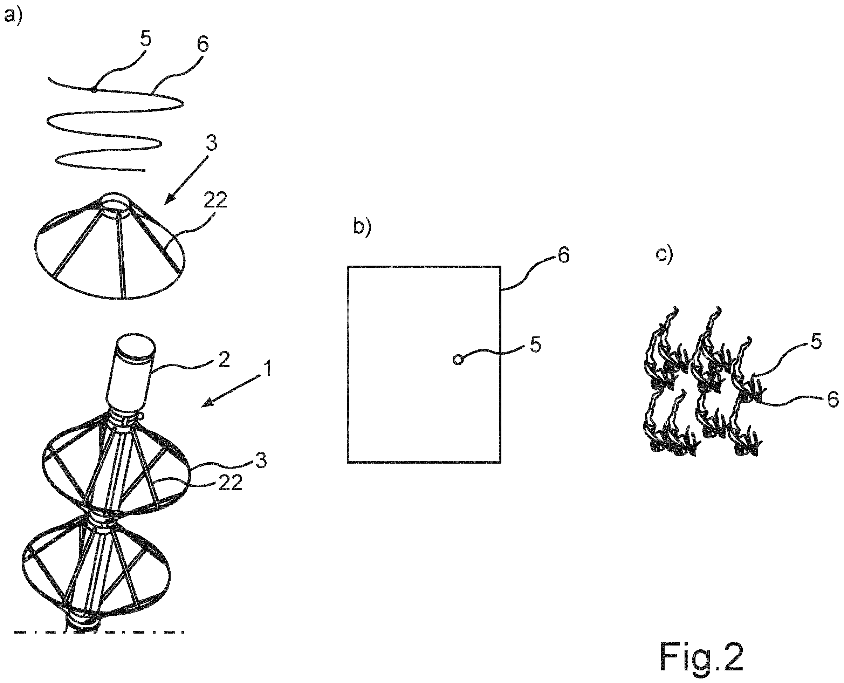

In Ergänzung zu Fig. 1 zeigt die Fig. 2 in den Teilansichten a), b) und c) unterschiedliche Ausgestaltungen von Vegetationselementen 6, wie sie mit verschiedenen Tragelementen 3, wie sie in Fig. 1 in den Teilansichten a), b) und c) gezeigt sind, verwendet werden können. Die in Fig. 2 a), b), c) gezeigten Vegetationselemente 6 lassen sich auf geeignete Weise an den jeweiligen gezeigten Tragelementen 3 befestigen (Fig. 1a)), auf diese aufbringen (Fig. 1b)) oder werden von diesen in der Bewegung begrenzt (Fig. 1c)). Gemäß der in Fig. 2a) gezeigten Ausführungsform wird das Vegetationselement 6 von einer Leine gebildet, die mit Sporen der zur Aufzucht vorgesehenen Algenspezies beschichtet oder imprägniert ist. Die entsprechende Leine lässt sich auf vorteilhafte Weise an einem Tragelement 3, wie es auch in Fig. 1a) gezeigt ist, mit einzelnen Streben 22, die eine kegelstumpfartige Mantelfläche aufspannen, befestigen. Bevorzugt wird die Leine um die einzelnen Streben 22 entlang der kegelstumpfförmigen Außenkontur des Tragelements 3 gewickelt und gespannt, sodass eine vergleichsweise große, dreidimensional geformte Vegetationsfläche 5 zur Aufzucht von Algen 4 bereitgestellt wird. Um die Leine an einem Tragelement 3 zu befestigen, wird zunächst ein Ende der Leine an einer Anschlussstruktur des Tragelements 3 befestigt und dann die Zentralstrebe 2 mit dem Tragelement 3 und der daran befestigten Leine gedreht. Das lose Ende der Leine wird schließlich ebenfalls zumindest mittelbar an dem Tragelement 3 befestigt oder fixiert. Alternativ wird zunächst die Leine auf das Tragelement 3 aufgewickelt und gespannt und erst dann das Tragelement 3 gemeinsam mit der daran befestigten Leine an der Zentralstrebe 2 befestigt.

Weiterhin zeigt Fig. 2a) in einer Detailansicht eine erfindungsgemäße Vorrichtung 1 zur Aufzucht von Algen in einem Gewässer 10, bei der eine Mehrzahl von einzelnen Tragelementen 3 modulartig zusammengesetzt und so ein vergleichsweise großes Tragelement 3 bilden. Gemäß der gezeigten Ausführungsform sind hierbei zwei der in Fig. 1a) gezeigten Tragelemente 3 an einer Zentralstrebe 2 befestigt. In Abhängigkeit der Wassertiefe, der Größe der zur Verfügung stehenden Gewässeroberfläche sowie der geplanten Aufzuchtleistung ist es durch Kombination einer Mehrzahl derartiger Tragelemente 3 oder Tragelementmodulen, die an einer Zentralstrebe 2 befestigt werden, möglich, die Produktionsleistung nahezu beliebig zu steigern und somit eine Anlage 24 zur Aufzucht von Algen 4 hinsichtlich ihrer Produktionsleistung zu skalieren.

Demgegenüber zeigt die Ausführungsform gemäß Fig. 2b) flächenförmiges Vegetationselement 6, dessen die Vegetationsfläche 5 bildende Oberfläche mit Sporen der gewünschten Algenspezies beschichtet oder imprägniert ist. Ein derartiges Vegetationselement kann die Oberfläche des in Figur 1b) gezeigten plattenförmigen Tragelementes 3 bilden, wobei das Vegetationselement 6 an dem Tragelement 3 befestigt oder ebenfalls in Form einer Beschichtung auf das Tragelement 3 aufgebracht werden kann. Im Übrigen ist es denkbar, das Tragelement 3 mit dem darauf angeordneten Vegetationselement 6 zu verformen, beispielsweise zu knicken, zu falten, zu biegen oder aufzurollen, insbesondere um das Verhältnis von Vegetationsfläche 5 zu benötigtem Raum zu maximieren.

Weiterhin ist es denkbar, dass ein plattenförmiges Tragelement 3 oder eine Mehrzahl von plattenförmigen Tragelementen 3 mit den Fig. 2b) gezeigten Vegetationselementen 6 verbunden und an einer Zentralstrebe 2 befestigt wird.

Fig. 2c) zeigt eine weitere spezielle Ausgestaltung von Vegetationselementen 6. Gemäß dieser Ausführungsform handelt es sich bei den Vegetationselementen 6 um Leinenfragmente, die mit Algensporen beschichtet oder imprägiert sind, oder um kleine Algenfragmente, also um kleine Algen oder Algenteile. Diese Fragmente lassen sich im Inneren einen käfig- oder korbartigen Tragelements 3 anordnen, das den freien Bewegungsraum der Fragmente im Gewässer 10 zu den Seiten sowie nach oben und unten begrenzt.

Auch in diesem Fall ist das in Fig. 2c) in einer Schnittansicht dargestellte Tragelement 3 wiederum an der erfindungsgemäß vorgesehenen Zentralstrebe 2 befestigt, wobei sich die als Algenfragmente ausgebildeten Vegetationselemente 6 während der Aufzucht freischwimmend im Inneren des käfig- oder korbartigen Tragelementes 3 zwischen diesem und der Zentralstrebe 2 befinden.

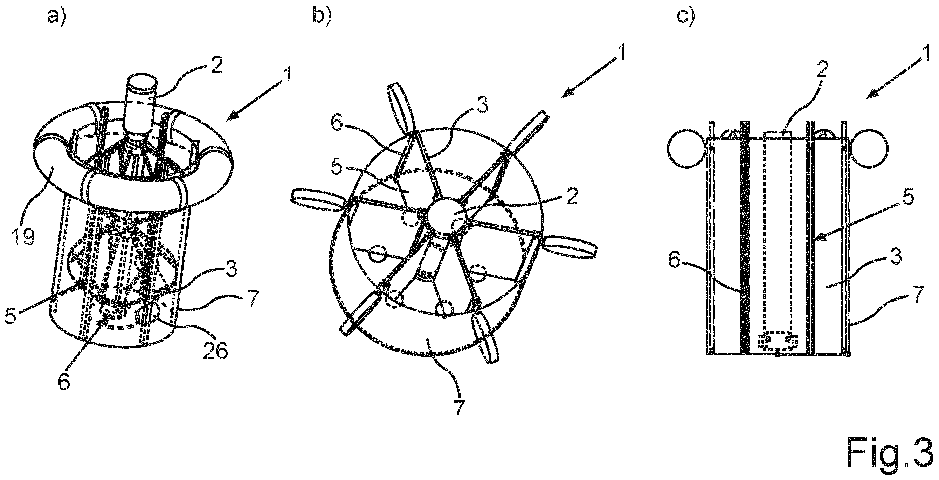

Im Weiteren ist es denkbar, wie in Fig. 3 gezeigt, zusätzlich zur Zentralstrebe 2 und dem damit verbundenen oder verbindbaren Tragelement 3 eine Abschirmeinheit 7 als äußere Begrenzungsfläche vorzusehen, die die Vorrichtung 1 am Außenumfang umgibt und gegenüber dem umgebenden Gewässer 10 begrenzt. Mithilfe einer derartigen Abschirmeinheit 7 ist es möglich, die Durchströmung des Vegetationsbereiches zu begrenzen, ein Vertreiben von Algenstücken, Sporen, Nährstoffen und/oder Mineralstoffen zu vermeiden oder zumindest zu minimieren und/oder die Wachstumsbedingungen, insbesondere die Lichtverhältnisse im Vegetationsbereich gezielt zu beeinflussen bzw. zu verändern.

Die Teilansichten a), b) und c) der Fig. 3 zeigen verschiedene Abschirmeinheiten 7 zur äußeren Begrenzung der Vegetationsflächen und deren Verwendung gemeinsam mit den unterschiedlich ausgebildeten, zuvor bereits beschriebenen Trag- und Vegetationselementen 3, 6. Hierbei ist es bei den in Fig. 3 gezeigten Ausführungsformen auf vorteilhafte Weise möglich, die Zentralstrebe 2 mit dem damit verbundenen Tragelement 3 in die Abschirmeinheit 7, die hier hohlzylinderförmig ausgeführt ist, einzusetzen und damit zu verbinden, sodass eine Baueinheit erzeugt wird. Diese Baueinheit kann selbst bereits schwimmfähig ausgeführt sein oder es ist, wie in Fig. 3 a), b) und c) gezeigt, ein Schwimmkörper 19, bevorzugt ein luftgefüllter Ring oder ein Ring aus einem schwimmfähigen Material, wie etwa Styropor oder einem Schaumstoff, vorgesehen, der zumindest mittelbar mit der Zentralstrebe 2, beispielsweise als Teil der Abschirmeinheit 7, befestigt ist. Diese Vorrichtung lässt sich dann über ein Befestigungselement 21, etwa einen Ring, eine Öse und/oder eine Klampe, die an der Zentralstrebe 2 angeordnet ist, zumindest mittelbar mit einer im Gewässeruntergrund angeordneten Verankerung 11 und/oder einer Schwimmleine 25 verbinden.

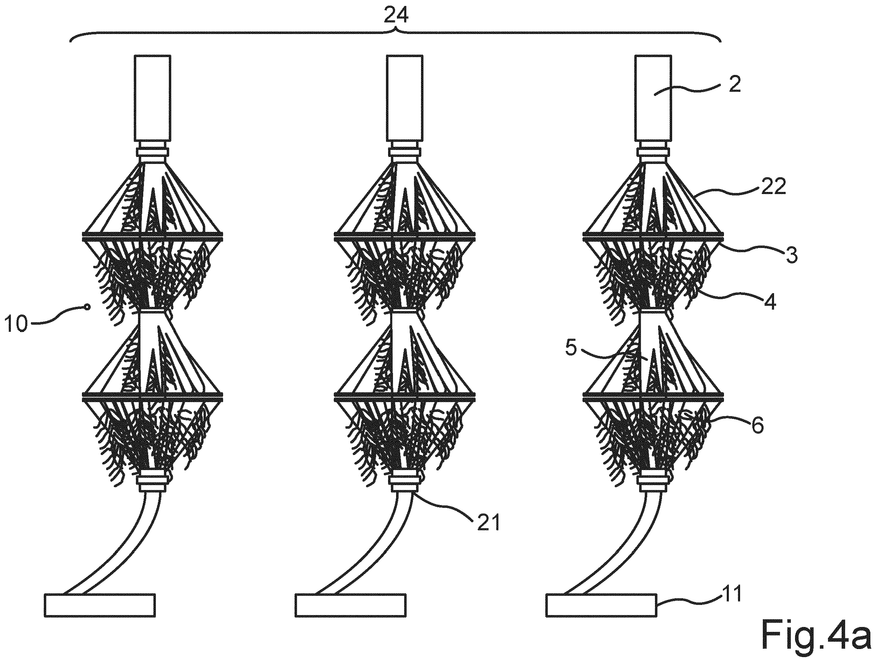

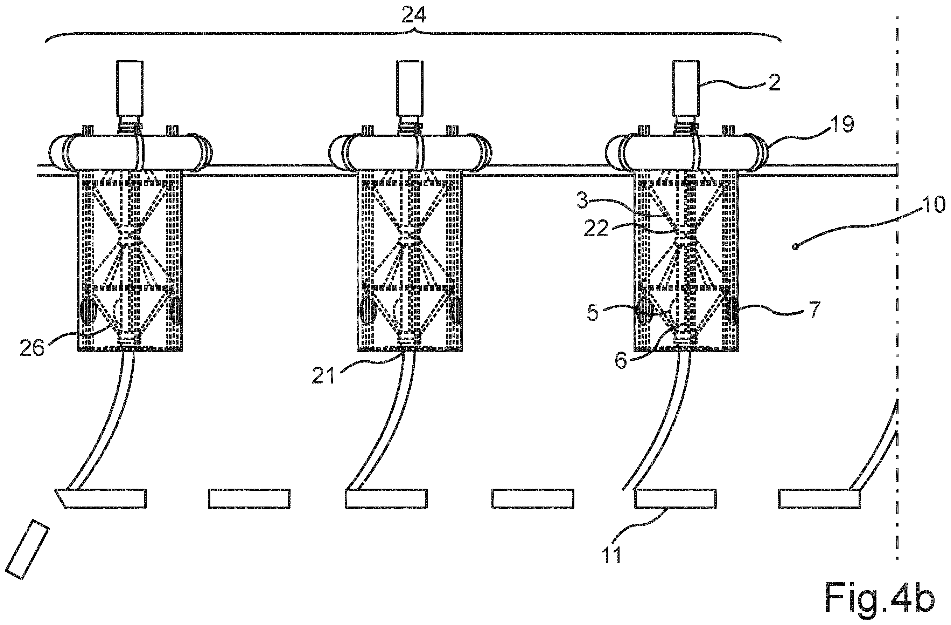

Fig. 4 zeigt in den Teilansichten a) und b) jeweils zumindest einen Teil von erfindungsgemäß ausgeführten Anlagen 24 zur Aufzucht von Algen 4 in einem Gewässer 10 mit einer Mehrzahl von Vorrichtungen 1, die gemäß der Erfindung ausgeführt sind.

Gemäß Fig. 4a) verfügen die Vorrichtungen 1 über keine Abschirmeinheit 7, während die in Fig. 4b) gezeigten Vorrichtungen 1 jeweils über eine Abschirmvorrichtung 7 verfügen. Im Übrigen weisen die in Fig. 4 dargestellten Vorrichtungen 1 jeweils eine Zentralstrebe 2, ein Tragelement 3, das aus mehreren Modulen besteht, und in Fig. 4b) einen Schwimmkörper 19 auf. Die Schwimmkörper 19 gemäß Fig. 4b) sind im oberen Bereich an der käfig- oder korbförmigen Abschirmeinheit 7 befestigt. Auf diese Weise wird sicherstellt, dass die Zentralstrebe 2 mit den daran befestigten Komponenten während des Aufenthalts in einem Gewässer 10 in der gewünschten Stellung verbleibt, sodass sich insbesondere die Tragelemente 3 mit den daran befestigten oder angeordneten Vegetationselementen 6 in der gewünschten Tiefe unterhalb der Wasseroberfläche befinden und stets mit Wasser bedeckt sind.

Gemäß der in Fig. 4b) gezeigten Ausführungsform haben die Schwimmkörper 19 einen Durchmesser von 0,8 m, die Höhe vom unteren Ende der Abschirmeinheit 7 mit einzelnen Öffnungen 26 in der Außenwand bis zum oberen Ende des Schwimmkörpers 19 beträgt 1 m und für den Mittenabstand zwischen benachbarten, zumindest mittelbar im Gewässeruntergrund verankerten Vorrichtungen 1 wurde ein Wert von 4 m gewählt.

Die einzelnen Zentralstreben 2 der in Fig. 4 gezeigten Vorrichtungen verfügen am unteren Ende über Befestigungselemente 21 zur Befestigung von Leinen oder Ketten und damit zur mittelbaren Befestigung der einzelnen Vorrichtungen und Verankerung im Gewässeruntergrund.

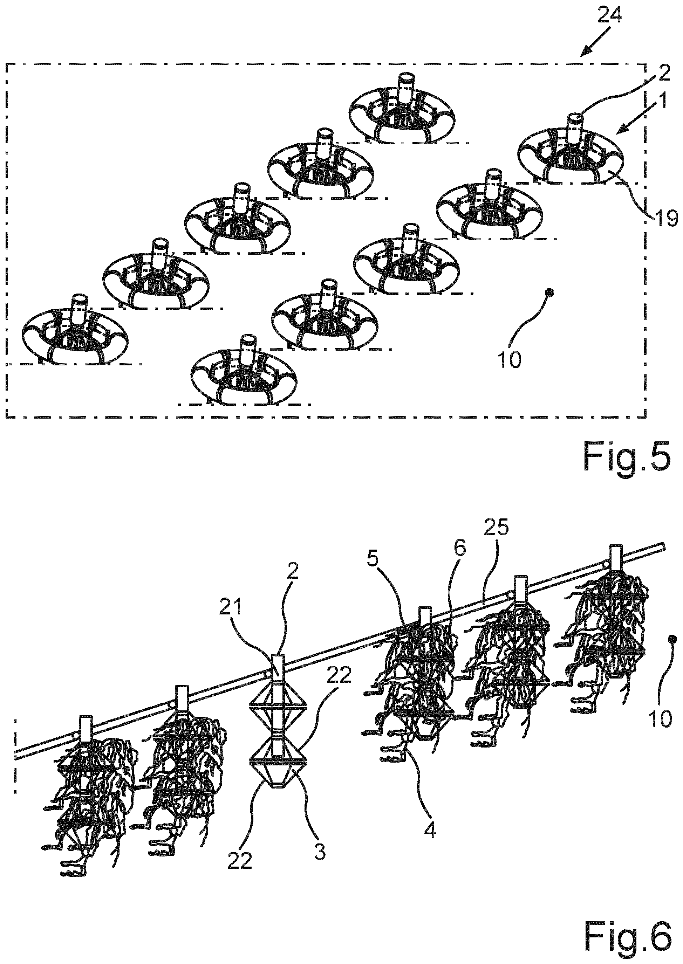

Ergänzend zeigt Fig. 5 eine schräge Draufsicht auf eine erfindungsgemäß ausgeführte Anlage 24 mit einer Mehrzahl von Vorrichtungen 1 zur Aufzucht von Algen, wie sie gemäß der in Fig. 4b) gezeigten Ausführungsform ausgebildet sind. Deutlich zu erkennen sind insbesondere die einzelnen Zentralstreben 2, die mit ihrem oberen Ende über die Wasseroberfläche des Gewässers hinausragen sowie die ringförmigen Schwimmkörper 19, die zumindest mittelbar über die Abschirmeinheit 7 an der Zentralstrebe 2 befestigt sind.

Ergänzend zeigt Fig. 6 eine weitere mögliche Anordnung einer Mehrzahl von erfindungsgemäß ausgebildeten Vorrichtungen 1, bei der die Zentralstreben 2 mit den daran befestigten, modulartig ausgebildeten Tragelementen 3 über im oberen Bereich der Zentralstreben 2 vorgesehenen Befestigungselementen 21 an einer Schwimmleine 25 befestigt sind.

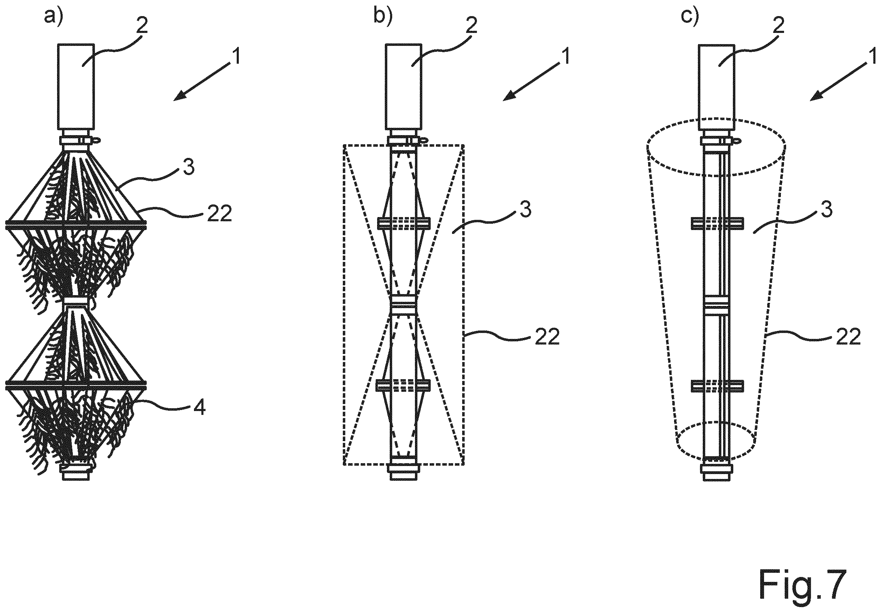

Im Weiteren zeigt Fig. 7 in den Teilansichten a), b) und c) unterschiedliche Ausgestaltungen von Tragelementen 3, die an einer Zentralstrebe 2 befestigt sind. Die Tragelemente 3 können jeweils modulartig also aus mehreren, bevorzugt baugleichen Modulen, wie in Fig. 7 a) gezeigt, oder einteilig ausgeführt sein. Die in Fig. 7 gezeigten Tragelemente 3 verfügen jeweils über einzelne Streben 22, die in der Mantelfläche des Tragelements 3 liegen und um die Vegetationselemente 6 in Form von Leinen aufgewickelt und gespannt werden können. Diese Leinen können wiederum mit Algensporen beschichtet oder imprägniert sein.

Alternativ ist es denkbar, die einzelnen Tragelemente 3 auf ihrer Oberfläche mit einer geeigneten Beschichtung als Vegetationselement 6 zu versehen, die dann die zur Aufzucht von Algen geeignete Vegetationsfläche 5 zur Verfügung stellt.

Es wird deutlich, dass die über Streben 22 verfügenden Tragelemente 3 unterschiedliche Körperformen haben können. Beispielhaft wird in Fig. 7a) ein Tragelement 3 in Form zweier übereinander angeordneter Polyeder mit zu den gegenüberliegenden Enden symmetrisch verlaufenden und zur Zentralstrebe 2 geneigneten Streben 22 gezeigt, die insgesamt vier kegelstumpfartige Mantelflächen bilden. Demgegenüber zeigt Fig. 7b) ein aus Streben 22 gebildetes Tragelement 3, das eine zylinderförmige Mantelfläche aufspannt und in Fig. 7c) ist ein eine kegelstumpfförmige Mantelfläche aufspannendes Tragelement 3 dargestellt.

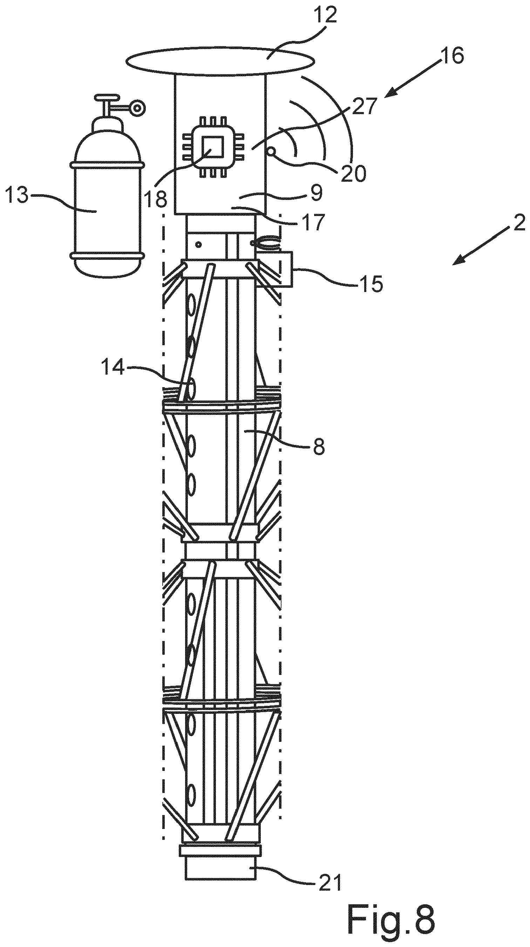

Fig. 8 zeigt eine besondere Ausführungsformen einer für eine erfindungsgemäße Vorrichtung 1 verwendbaren Zentralstrebe 2. Gemäß der gezeigten Ausführungsform verfügt die Zentralstrebe 2 über eine Mehrzahl von Funktionseinheiten. So kann eine Zentralstrebe 2 bedarfsgerecht ausgestattet werden und etwa einen Kohlenstoffdioxidspeicher 13 mit einer Dosiereinheit 14 in oder an der Zentralstrebe 2 vorgesehen sein, sodass eine gezielte Kohlenstoffdioxidausbringung in einen die Zentralstrebe 2 umgebenden Bereich möglich ist. Weiterhin kann es sinnvoll sein, wenigstens einen geeigneten Sensor 15 vorzusehen, der insbesondere die Wassertemperatur, Wasserzusammensetzung, den Kohlenstoffdioxidgehalt im Wasser, die Lichtintensität über und/oder unter der Wasseroberfläche und/oder Strömungsparameter erfasst. Im oberen Bereich der Zentralstrebe 2, der sich während der Algenaufzucht vorzugsweise oberhalb der Wasseroberfläche befindet, sind auf vorteilhafte Weise weitere elektronische Komponenten, wie eine elektronische Datenspeicher- und/oder -verarbeitungseinheit 17, 18 vorgesehen. Ebenso lässt sich eine derartige Zentralstrebe 2 mit einem Funkmodul 20 und/oder einer Datenschnittstelle 27 ausrüsten, die eine uni- oder bidirektionale Datenkommunikation mit einer zentralen Datenverarbeitungseinheit, die an Land, auf einer Plattform oder auf einem Schiff angeordnet sein kann, ermöglicht.

Ebenso ist es denkbar, zur Versorgung der einzelnen Funktionseinheiten der Zentralstrebe 2 ein Fotovoltaikmodul 12 und/oder einen Energiespeicher 9 vorzusehen, sodass die Zentralstrebe 2 und ihre Funktionseinheiten, insbesondere auch eine Leuchteinheit 8, die in der Zentralstrebe 2 zur Beleuchtung der Algen 4 angeordnet ist, ohne Anbindung an eine externe Energiequelle betreibbar sind.

Mittels einer in oder an der Zentralstrebe 2 vorgesehenen Leuchteinheit 8, die insbesondere UV-Licht emittiert und bevorzugt in Form einer LED ausgebildet ist, können die Algen 4 auch bei Nacht oder Dunkelheit aus sonstigem Grund auf geeignete Weise beleuchtet werden. In Kombination mit einem Fotovoltaikmodul 12 das elektrische Energie erzeugt, die in einem Energiespeicher 9 zwischengespeichert wird, kann eine besonderes effektive Algenaufzucht realisiert werden.

Durch den Einsatz von künstlichem Licht in der Tiefe, können Algen 4 auch dort angebaut werden, wo sie sonst nicht oder nur langsam wachsen würden. Außerdem wird durch die höhere Lichtintensität das normale Wachstum erhöht. Es kann damit auch in Monaten mit sehr geringer Tageslichteinstrahlung wirtschaftlich angebaut werden. Im Weiteren bietet die erfindungsgemäß ausgebildete Vorrichtung 1 den Vorteil, dass durch die Bereitstellung dreidimensionaler Anbauflächen ein vergleichsweise hoher Ertrag pro genutzter Gewässerroberfläche erzielbar ist.

### Bezugszeichenliste

- 1: Vorrichtung zur Aufzucht von Algen
- 2: Zentralstrebe
- 3: Tragelement
- 4: Algen
- 5: Vegetationsfläche
- 6: Vegetationselement
- 7: Abschirmeinheit
- 8: Leuchteinheit
- 9: Energiespeicher
- 10: Gewässer
- 11: Verankerung
- 12: Fotovoltaikmodul
- 13: Kohlendioxidspeicher
- 14: Dosiereinheit
- 15: Sensor
- 16: Datensende- und/oder -empfangseinheit
- 17: Datenspeicher
- 18: Datenverarbeitungseinheit
- 19: Schwimmkörper
- 20: Funkmodul
- 21: Befestigungselement
- 22: Strebe
- 23: Anlage
- 24: Schwimmkörper
- 25: Schwimmleine
- 26: Öffnung
- 27: Datenschnittstelle

## Patentansprüche

1. Vorrichtung (1) zur Aufzucht und Weiterverarbeitung von Algen in einem Gewässer sowie zur Wasserfiltration (10) mit einem Grundkörper und einem Tragelement (3), das eine zur Kultivierung von Algen (4) geeignete Vegetationsfläche (5) begrenzt und/oder an dem ein Vegetationselement (6) mit einer zur Kultivierung von Algen (4) geeigneten Vegetationsfläche (5) befestigbar oder befestigt ist, wobei der Grundkörper gemeinsam mit dem Tragelement (3) zumindest teilweise unter eine Wasseroberfläche des Gewässers (10) absenkbar ist,
**dadurch gekennzeichnet, dass** der Grundkörper eine Zentralstrebe (2) aufweist, durch die Zug- und Druckkräfte in Längsrichtung der Zentralstrebe (2) aufnehmbar sind, und dass das wenigstens eine Tragelemente (3) zumindest mittelbar an der Zentralstrebe (2) befestigt und zumindest bereichsweise beabstandet zu einer Außenfläche der Zentralstrebe (2) angeordnet ist.

2. Vorrichtung nach Anspruch 1,
**dadurch gekennzeichnet, dass** Tragelement (3) wenigstens ein Seil, eine Leine und/oder eine Kette aufweist, dessen/deren Längsachse parallel oder in einem spitzen Winkel zur Außenumfangsfläche der Zentralstrebe (2) angeordnet ist.

3. Vorrichtung nach Anspruch 2,
**dadurch gekennzeichnet, dass** das Tragelement (3) aus wenigstens zwei Modulen und/oder Segmenten gebildet wird.

4. Vorrichtung nach einem der vorangehenden Ansprüche,
**dadurch gekennzeichnet, dass** das Vegetationselement (6) als Schicht auf dem Tragelement (3) ausgeführt ist.

5. Vorrichtung nach einem der vorangehenden Ansprüche,
**dadurch gekennzeichnet, dass** das Tragelement (3) netz- und/oder maschenartig ausgebildet ist.

6. Vorrichtung nach einem der vorangehenden Ansprüche,
**dadurch gekennzeichnet, dass** das Tragelement (3) wenigstens abschnittsweise in Form eines Hohlzylinders, Hohlkegelstumpfs und/oder Hohlquaders ausgebildet ist.

7. Vorrichtung nach einem der Vorangehenden Ansprüche,
**dadurch gekennzeichnet, dass** das Tragelement (3) ein Plattenelement aufweist, das in einer Ebene liegt, die parallel zur Längsachse der Zentralstrebe (2) angeordnet ist.

8. Vorrichtung nach einem der vorangehenden Ansprüche,
**dadurch gekennzeichnet, dass** das Tragelement (3) zwischen der Zentralstrebe (2) und einer umgebenden, bevorzugt hohlzylinder- oder hohlquaderförmig ausgebildeten Abschirmeinheit (7) angeordnet ist.

9. Vorrichtung nach einem der vorangehenden Ansprüche,
**dadurch gekennzeichnet, dass** die Zentralstrebe (2) eine Leuchteinheit (8) aufweist.

10. Vorrichtung nach Anspruch 9,
**dadurch gekennzeichnet, dass** die Leuchteinheit (8) über wenigsten eine LED verfügt, die geeignet ist, Licht mit einer Wellenlänge von 100 bis 380 nm zu emittieren.

11. Vorrichtung nach einem der vorangehenden Ansprüche,
**dadurch gekennzeichnet, dass** die Zentralstrebe (2) über wenigstens einen Lichtleiter und/oder ein Element zur Umlenkung von Strahlung verfügt.

12. Vorrichtung nach einem der vorangehenden Ansprüche,
**dadurch gekennzeichnet, dass** die Zentralstrebe (2) wenigstens einen Energiespeicher (9) zur Speicherung von elektrischer Energie aufweist.

13. Vorrichtung nach einem der vorangehenden Ansprüche,
**dadurch gekennzeichnet, dass** Mittel zur wenigstens mittelbaren Verankerung (11) in einem Gewässergrund vorgesehen sind.

14. Vorrichtung nach einem der vorangehenden Ansprüche,
**dadurch gekennzeichnet, dass** die Zentralstrebe (2) wenigstens ein Fotovoltaikmodul (12), einen Kohlenstoffdioxidspeicher (13), eine Dosiereinheit (14) zur Ausbringung von Kohlenstoffdioxid in das Gewässer (10), einen Sensor (15), eine Datensende- und/oder -empfangseinheit (16), einen Datenspeicher (17) und/oder eine Datenverarbeitungseinheit (18) aufweist.

15. Anlage (24) zur Algenaufzucht in einem Gewässer (10) mit einer Mehrzahl von Vorrichtungen (1) gemäß einem der vorangehenden Ansprüche, die wenigstens teilweise in einem Gewässeruntergrund verankert und/oder miteinander verbunden sind.
